# FASCICULE DE BREVET EUROPEEN

(11) **EP 3 487 386 B1**
(45) Date de publication et mention de la délivrance du brevet: **07.07.2021**
(21) Numéro de dépôt: 17754404.6
(22) Date de dépôt: 21.07.2017
(51) Int. Cl.: A61B 5/00, A61B 18/20

(54) **SYSTÈME DE DIAGNOSTIC ET DE TRAITEMENT DERMATOLOGIQUE**
DERMATOLOGISCHES DIAGNOSE- UND BEHANDLUNGSSYSTEM
DERMATOLOGICAL DIAGNOSIS AND TREATMENT SYSTEM

(30) Priorité: 22.07.2016 FR 1657051
(43) Date de publication de la demande: 29.05.2019
(73) Titulaire: Université de Bordeaux, 33000 Bordeaux (FR); Centre National de la Recherche Scientifique, 75016 Paris (FR); Irisiome, 33400 Talence (FR); Institut d'Optique Graduate School, 91127 Palaiseau (FR); Commissariat à l'Energie Atomique et aux Energies Alternatives, 75015 Paris (FR)
(72) Inventeur: ROYON, Romain, 40100 Dax (FR); SANTARELLI, Giorgio, 33400 Talence (FR); LHERMITE, Jérôme, 64600 Anglet (FR); DUBRASQUET, Romain, 40630 Sabres (FR)
(74) Mandataire: Jacobacci Coralis Harle
(86) Numéro de dépôt international: PCT/FR2017/052012
(87) Numéro de publication internationale: WO 2018/015689

(56) Documents cités:
- US-A1- 2001 047 137
- US-A1- 2001 056 237
- US-A1- 2007 197 883
- US-A1- 2009 227 994
- US-A1- 2010 010 480

## Description

### DOMAINE TECHNIQUE AUQUEL SE RAPPORTE L'INVENTION

La présente invention concerne de manière générale le domaine des appareils de diagnostic et/ou de traitement dermatologique.

Elle concerne plus particulièrement un système de diagnostic optique et de traitement laser appliqué à la dermatologie.

Elle concerne en particulier un appareil de diagnostic et de traitement pour la modification de chromophores cutanés, par exemple pour la suppression de tatouages.

### ARRIERE-PLAN TECHNOLOGIQUE

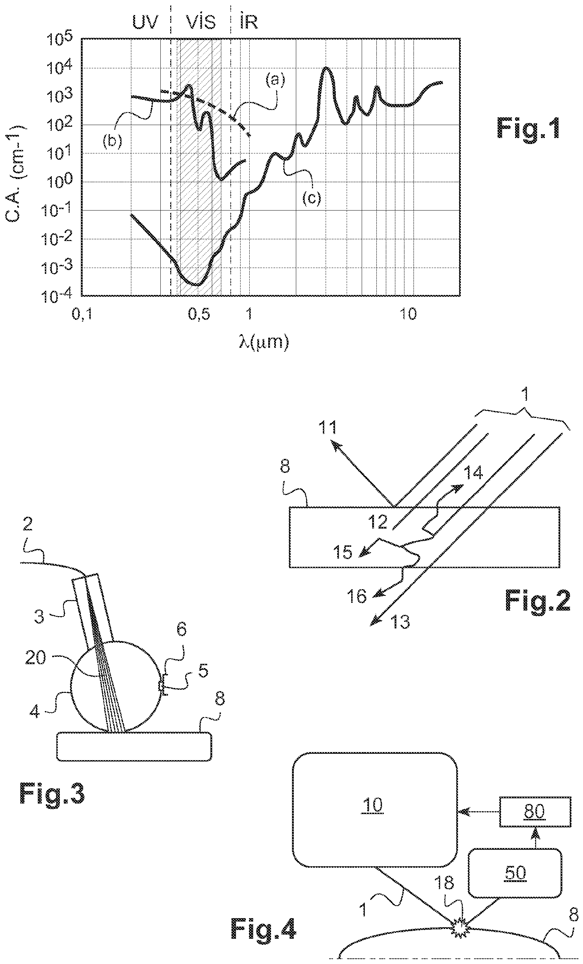

Les traitements dermatologiques par laser sont en général basés sur l'absorption d'une onde laser par différents chromophores cutanés. Certains chromophores sont endogènes comme la mélanine, l'hémoglobine et l'eau. D'autres chromophores sont exogènes comme les pigments des tatouages. Lorsqu'une énergie suffisante est absorbée par un chromophore, celui-ci est détruit et éliminé de manière naturelle par des processus physiologiques mettant en œuvre des macrophages. La figure 1 représente des mesures spectroscopiques de coefficient d'absorption (CA) des principaux chromophores des tissus épidermiques, respectivement la mélanine en (a) sur la figure 1, l'hémoglobine en (b) et l'eau en (c). Les mesures de coefficient d'absorption sont ici représentées en fonction de la longueur d'onde (λ) dans le domaine spectral s'étendant de l'ultra-violet (UV, de 100 nm à 350 nm), au visible (VIS, de 350 nm à 800 nm) et au proche infrarouge (NIR, de 850 nm à 1,5 µm) ou même à l'infra-rouge lointain (IR de 1,5 µm à 10 µm) pour l'eau.

Le document US 2009/0227994 A1 décrit un dispositif pour éliminer un composé, par exemple un pigment de tatouage, d'un tissu de peau. Le document US 2001/0056237 A1 décrit un appareil et une méthode pour contrôler in vivo et de manière non invasive la présence d'un ou plusieurs chromophores dans un échantillon de tissu de peau, de tissu épithélial ou sous-épithélial. Le document US 2007/0197883 A1 décrit un appareil de diagnostic pour mesurer un spectre de réflexion diffuse ou de diffusion Raman de pigments de tatouage. Le document US 2010/010480 décrit un système et un procédé de délivrance d'énergie percutanée.

Actuellement, un appareil de traitement laser conventionnel en dermatologie utilise des impulsions dont la durée est comprise entre quelques centaines de picosecondes et plusieurs nanosecondes et de fortes énergies (de 200 mJ à plusieurs Joules). Les traitements dermatologiques par laser utilisent en général des effets thermiques qui peuvent générer des traumatismes post-opératoires importants. L'effet thermique n'étant pas spécifique, cet effet correspond à une brûlure par carbonisation des cibles sans respecter les tissus voisins. Ceci est dû au fait que l'onde laser utilisée ne correspond pas spécifiquement à un pic d'absorption et qu'il faut donc beaucoup d'énergie pour altérer la cible entraînant des dommages sur l'environnement immédiat. De nombreux traumatismes post-opératoires apparaissent comme une hyperpigmentation (peau plus sombre), une hypopigmentation (peau plus claire), une décoloration de la peau, une cicatrice et/ou une infection. Le traitement de ces effets secondaires s'effectue lors de la cicatrisation lorsque la peau se reconstitue.

Or, le choix de la longueur d'onde utilisée pour un traitement laser est primordial en dermatologie. En effet, l'absorption et la pénétration de la lumière dépendent fortement de la longueur d'onde (Fig.1). La gamme complète de lasers disponibles sur le marché couvre un grand nombre de longueurs d'onde notamment grâce à l'arrivée des diodes lasers. Toutefois, si un praticien souhaite traiter un grand nombre de pathologies et un grand nombre de patients ayant des phototypes différents ou des tatouages différents, il doit disposer d'un parc laser important. Cela représente un investissement de plusieurs centaines de milliers d'euros avec un coût de maintenance d'environ 10% du prix d'achat par an. Enfin, le fait de ne disposer que d'un laser, comme c'est souvent le cas, ne permet pas d'effacer toutes les encres lors d'un traitement visant à supprimer un tatouage. Par conséquent, l'efficacité d'un traitement laser pour enlever un tatouage est mauvaise, notamment sur les tatouages en couleurs.

On connaît notamment du document US 2007/0197883 un appareil de diagnostic spectroscopique comme aide pour l'enlèvement de tatouage au laser. Cet appareil de diagnostic comporte un laser d'excitation et un spectromètre Raman, qui effectue une mesure d'absorption Raman sur la peau tatouée afin de déterminer les raies d'absorption Raman. L'appareil de diagnostic comporte en outre une source de lumière large bande et un spectromètre pour mesurer un spectre de réflectance diffuse. Le spectre Raman est utilisé pour déterminer la composition des pigments d'un tatouage. Le spectre de réflectance diffuse est utilisé pour estimer un taux d'absorption d'un laser d'enlèvement de tatouage à une longueur d'onde déterminée. Les spectres Raman et de réflectance diffuse sont utilisés pour sélectionner les paramètres d'un laser de détatouage avant ou pendant le traitement laser. L'appareil permet de sélectionner un laser de type Q-switch ayant une longueur d'onde, une énergie d'impulsion, une puissance crête, et une cadence adaptées pour le traitement laser d'enlèvement de tatouage de la peau analysée. Dans le présent document, la cadence équivaut à la fréquence de répétition des impulsions laser.

On connaît aussi du document WO 98/24514 une méthode et un appareil de traitement dermatologique comprenant un laser asservi à un capteur de température utilisé pour détecter un dommage de l'épiderme induit par le traitement laser. Selon ce document, une boucle d'asservissement agit sur l'énergie, la durée d'impulsion et/ou la cadence du laser afin d'éviter d'endommager l'épiderme.

Actuellement, il n'existe pas d'appareil de traitement dermatologique par laser capable de déterminer et d'adapter automatiquement les conditions de traitement optimales en temps réel.

Il existe un besoin pour un appareil de diagnostic et de traitement laser permettant d'enlever localement un pigment de tatouage sur un épiderme particulier sans risque d'endommager les tissus épidermiques environnants.

Il existe un besoin pour un appareil unique de diagnostic et de traitement laser permettant d'enlever différents pigments de tatouage sur différents types d'épidermes sans risque d'endommager les tissus épidermiques environnants.

### OBJET DE L'INVENTION

Afin de remédier à l'inconvénient précité de l'état de la technique, la présente invention propose un système de diagnostic et de traitement dermatologique comprenant un dispositif de source lumineuse, un dispositif de collecte de flux lumineux, un dispositif de mesure spectroscopique d'absorption et un calculateur.

Plus particulièrement, on propose selon l'invention un système de diagnostic et de traitement dermatologique dans lequel le dispositif de source lumineuse est adapté pour générer un premier faisceau lumineux de faible intensité (de 1 mW à 100 mW de puissance moyenne) en direction d'une surface épidermique d'un patient, le premier faisceau lumineux s'étendant sur une gamme spectrale ou étant accordable en longueur d'onde sur une gamme spectrale, le dispositif de collecte de flux lumineux étant arrangé pour collecter un premier flux lumineux par réflexion et/ou diffusion du premier faisceau lumineux sur ladite surface épidermique, le dispositif de mesure spectroscopique d'absorption étant adapté pour enregistrer une première mesure spectroscopique du premier flux lumineux en fonction de la gamme spectrale, le calculateur étant adapté pour recevoir la première mesure spectroscopique dans la gamme spectrale et pour déterminer une première longueur d'onde associée à une absorption d'un pigment de ladite surface épidermique et une deuxième longueur d'onde associée à une absorption d'un autre constituant tissulaire de ladite surface épidermique, et dans lequel le dispositif de source lumineuse comporte une source laser accordable en longueur d'onde à l'intérieur de la gamme spectrale, la source laser étant adaptée pour générer un deuxième faisceau laser de traitement de forte intensité (de 100 mW à 100 W de puissance moyenne) à ladite première longueur d'onde en direction de ladite surface épidermique.

Ce système non-invasif permet de traiter spécifiquement un pigment cutané pour le décomposer par absorption spectralement spécifique tout en limitant fortement l'échauffement du tissu épidermique environnant.

D'autres caractéristiques non limitatives et avantageuses du système de diagnostic et de traitement dermatologique conforme à l'invention, prises individuellement ou selon toutes les combinaisons techniquement possibles, sont les suivantes :
- le deuxième faisceau laser est constitué d'impulsions laser de durée comprise entre 500 femtosecondes (fs) et 100 microsecondes (µs) et, de préférence, comprise entre 30 picosecondes (ps) et 10 nanosecondes (ns) ;
- le deuxième faisceau laser est constitué d'impulsions à une cadence comprise entre 1 hertz (Hz) et 1 gigahertz (GHz) et, de préférence, comprise entre 10 mégahertz (MHz) et 500 MHz ;
- le deuxième faisceau laser a une puissance comprise entre 100 milliwatts (mW) et 100 W ;
- le dispositif de source lumineuse comporte une source laser continument accordable en longueur d'onde sur une gamme spectrale s'étendant au moins sur 100 nm, et de préférence de l'ultraviolet au proche infrarouge, la source laser étant adaptée pour générer le deuxième faisceau laser ;
- le dispositif de collecte de flux lumineux comporte une sphère intégrante, et le dispositif de mesure spectroscopique d'absorption comporte un spectromètre ou un monochromateur et un photo-détecteur ;
- le dispositif de collecte de flux lumineux est configuré pour collecter un deuxième flux lumineux par réflexion et/ou diffusion du deuxième faisceau lumineux sur ladite surface épidermique et le dispositif de mesure spectroscopique d'absorption est adapté pour enregistrer un signal de mesure du deuxième flux lumineux à la première longueur d'onde,
- le système de diagnostic et de traitement dermatologique comporte en outre un capteur de température arrangé pour enregistrer un signal de mesure de température de ladite surface épidermique en fonction de l'application du deuxième faisceau laser ;
- la source laser étant en outre accordable en durée et/ou en cadence, le système de diagnostic et de traitement dermatologique comporte en outre un dispositif de rétroaction configuré pour modifier la durée et/ou la cadence des impulsions laser en fonction du signal de mesure du deuxième flux lumineux et/ou en fonction du signal de mesure de température ;
- le dispositif de mesure spectroscopique comporte un détecteur d'image ayant une matrice de pixels adaptée pour former une image hyperspectrale de ladite surface épidermique dans ladite gamme spectrale, et le calculateur étant adapté pour déterminer la deuxième longueur d'onde de traitement laser correspondant à chaque pixel du détecteur d'image à partir de ladite image hyperspectrale.

Selon un mode de réalisation, le dispositif de source lumineuse comporte une autre source lumineuse adaptée pour générer le premier faisceau lumineux. L'autre source lumineuse est choisie de préférence parmi une lampe à lumière blanche, une diode électroluminescente continue ou puisée.

Selon un autre mode de réalisation, le dispositif de source lumineuse comporte une seule source laser accordable en longueur d'onde, la source laser étant adaptée pour générer le premier faisceau lumineux et le deuxième faisceau laser et le dispositif de source lumineuse comporte un dispositif de modulation en intensité du faisceau laser généré par la source laser.

L'invention propose également un procédé de diagnostic et de traitement dermatologique comprenant les étapes suivantes :
- génération d'un premier faisceau lumineux de faible intensité en direction d'une surface épidermique d'un patient,
- collecte d'un premier flux lumineux par réflexion et/ou diffusion du premier faisceau lumineux sur ladite surface épidermique,
- analyse spectroscopique du premier flux lumineux pour en déduire un enregistrement d'une première mesure spectroscopique,
- détermination, par traitement numérique de la première mesure spectroscopique, d'une première longueur d'onde associée à une absorption d'un pigment de ladite surface épidermique et d'une deuxième longueur d'onde associée à une autre absorption d'un tissu de ladite surface épidermique, et
- ajustement d'une source d'impulsions laser accordable en longueur d'onde à la première longueur d'onde et génération d'un deuxième faisceau laser de traitement de forte intensité en direction de ladite surface épidermique à ladite première longueur d'onde.

### DESCRIPTION DETAILLEE D'UN EXEMPLE DE REALISATION

La description qui va suivre en regard des dessins annexés, donnés à titre d'exemples non limitatifs, fera bien comprendre en quoi consiste l'invention et comment elle peut être réalisée.

Sur les dessins annexés :
- la figure 1 représente des mesures spectroscopiques de coefficient d'absorption (CA) des principaux chromophores des tissus épidermiques ;
- la figure 2 représente schématiquement les différents phénomènes physiques d'interaction entre un faisceau laser et un tissu épidermique ;
- la figure 3 représente schématiquement une vue en coupe d'un dispositif de mesure spectroscopique en réflexion ou rétrodiffusion;
- la figure 4 représente schématiquement un système de diagnostic et de traitement selon un mode de réalisation ;
- la figure 5 représente schématiquement un procédé du traitement selon un mode de réalisation ;
- la figure 6 représente schématiquement une variante du procédé du traitement selon un mode de réalisation ;
- la figure 7 représente un diagramme de gravité de brûlure en fonction du temps de contact et de la température.

### Dispositif

Un aspect de la présente divulgation consiste à déterminer dans quelle proportion la peau reflète un rayonnement lumineux de manière à déterminer précisément la longueur d'onde du traitement laser la plus adaptée au pigment à décomposer et à la peau du patient.

De manière générale, lorsqu'un faisceau lumineux incident 1 atteint la surface d'un tissu épidermique 8, une partie du faisceau incident forme un faisceau réfléchi 11. Le reste du faisceau incident se propage dans le tissu épidermique 8. Une autre partie 12 est absorbée par les constituants tissulaires. Encore une autre partie du faisceau incident est diffusée vers l'arrière (faisceau rétro-diffusé 14), vers l'avant (faisceau diffusé vers l'avant 16) et dans le tissu épidermique (faisceau de diffusion latérale 15). Enfin, une dernière partie faisceau incident peut parvenir à traverser le volume tissulaire et former un faisceau transmis 13.

La réflexion optique à la surface du tissu limite l'efficacité du traitement laser. En fonction de la longueur d'onde et de la nature de la surface des tissus 8, la réflexion optique peut être spéculaire ou diffuse. La réflexion en surface peut atteindre des valeurs élevées. On évalue à environ 30% le coefficient de réflexion d'un faisceau laser sur la peau à une longueur d'onde de 488 nm. La diffusion est une interaction de la lumière avec la matière au cours de laquelle la direction du faisceau incident est modifiée. Le faisceau rétrodiffusé 14 ressort du tissu 8. Par contre, le faisceau absorbé 12 et les faisceaux diffusés 15, 16 transforment l'énergie apportée par les photons incidents en chaleur.

La proportion des phénomènes de réflexion, absorption, transmission et diffusion dépend, pour un tissu donné, de la longueur d'onde du faisceau incident. Dans l'ultraviolet court et dans l'infrarouge lointain, l'absorption est plus importante que la diffusion. Dans les autres parties du spectre (de 300nm à 2µm), la diffusion influence la géométrie du faisceau en produisant un élargissement latéral et en diminuant la profondeur de pénétration du faisceau laser.

Sur la figure 3, est représenté un dispositif de mesure spectroscopique d'absorption. Ce dispositif comporte un dispositif de source lumineuse 2, un système optique 3, un dispositif de collecte 4 du flux lumineux réfléchi et rétro-diffusé, un dispositif de spectrométrie 6 et un dispositif de photo-détection 5.

Le dispositif de source lumineuse 2 émet un faisceau lumineux 20 de faible intensité (de 1 mW à 100mW de puissance moyenne). On peut utiliser un dispositif de source lumineuse 2 émettant sur un spectre large, bande spectrale couvrant une gamme spectrale pouvant aller jusqu'à 100 nm. A titre d'exemple, le dispositif de source comporte un laser supercontinuum. Toutefois, il suffit généralement d'effectuer des mesures à quelques longueurs d'onde discrètes dans le domaine spectral. Dans un autre exemple, le dispositif de source comporte plusieurs diodes lasers de faible puissance moyenne (de 1 mW à 100 mW) adaptées pour choisir différentes longueurs d'onde dans plusieurs bandes spectrales correspondant à différentes fenêtres thérapeutiques (visible : 500-700 nm, infra-rouge : autour de 1 micromètre). Le système optique 3 sert à mettre en forme le faisceau lumineux incident et à adapter sa taille en fonction de la fluence voulue.

Ce dispositif de source lumineuse 2 permet d'éclairer le tissu épidermique 8 à traiter sous un faible flux lumineux de manière à éviter d'altérer le tissu épidermique et de changer les propriétés optiques de réflexion, d'absorption, de diffusion et de transmission du tissu épidermique.

Le dispositif de collecte 4 permet de collecter le flux lumineux réfléchi ou rétrodiffusé par la peau. Le dispositif de collecte 4 est configuré de manière à collecter un maximum de photons sous éclairement d'une source lumineuse 2 de faible intensité. A titre d'exemple, le dispositif de collecte 4 comprend une sphère intégrante.

Le dispositif de spectrométrie 6 comporte par exemple un spectromètre équipé d'un détecteur 5 configuré pour opérer dans le domaine spectral de la source lumineuses 2 en fonction des longueurs d'onde d'intérêt. Dans un autre exemple, le dispositif de spectrométrie 6 comprend un monochromateur équipé d'un détecteur 5, qui analyse directement une longueur d'onde réfléchie par la peau du patient.

Le dispositif de spectrométrie 6 et de photodétection 5 peut être intégré au dispositif de collecte 4. De façon alternative, le dispositif de spectrométrie 6 et de photodétection 5 est relié par fibre optique au dispositif de collecte 4, de manière à déporter le boîtier du dispositif de spectrométrie 6 et/ou de photodétection 5.

Le dispositif de mesure spectroscopique d'absorption est étalonné lors de la fabrication. On effectue de préférence une calibration avant chaque utilisation ultérieure. La source de calibration peut être le laser lui-même fixé à une longueur d'onde donnée ou une source supplémentaire ajoutée dans la machine (LED ou diode laser). Dans un premier cas, la calibration consiste à disposer un miroir face à une ouverture de la sphère intégrante pour mesurer l'intensité lumineuse maximum réfléchie. Dans un autre exemple, la calibration consiste à disposer un puits de lumière. Les mesures de réflexion sur un tissu épidermique sont ensuite rapportées à cette intensité lumineuse maximum, pour obtenir des mesures spectroscopiques relatives. L'absorption se calcule alors en soustrayant la mesure en réflexion de référence (avec le miroir) à la mesure en réflexion sur le tissu épidermique.

La figure 4 représente un système de diagnostic et de traitement selon un premier mode de réalisation. Le système comporte un dispositif de source laser de traitement 10, un dispositif de détection 50 et un dispositif de rétroaction 80. Le dispositif de source laser 10 émet un faisceau laser 1 de traitement en direction de la peau 8 à traiter. Dans un exemple de réalisation, le faisceau laser 1 est continu temporellement. De préférence, le faisceau laser 1 est un faisceau impulsionnel avec des impulsions de durée comprise entre 30 ps jusqu'à 10 ns. La cadence des impulsions laser peut varier entre 500 MHz et 500 kHz. Ce domaine de fonctionnement temporel permet d'utiliser une impulsion laser ayant à la fois une puissance crête élevée et une puissance moyenne relativement importante. Cette impulsion laser permet de combiner des effets photo-ablatifs couplés à des effets thermiques de façon particulièrement innovante en dermatologie. Cette sélection d'un domaine temporel de durée et de cadence des impulsions laser ouvre la voie à une nouvelle sorte d'interaction dans laquelle les effets photo-ablatifs et les effets thermiques peuvent jouer des rôles complémentaires. Cette source est basée sur une architecture laser dite à mode bloqué à haute cadence (entre 10 MHz et 500 MHz). Le régime mode bloqué peut être obtenu par l'utilisation d'un absorbant saturable, d'un modulateur ou de rotation non linéaire de la polarisation. L'association de cette source à un élément permettant d'extraire une ou plusieurs impulsions (modulateur acousto optique ou electro-optique, cellule de pockels) génère une impulsion unique ou des paquets de plusieurs impulsions à cadence variable. La durée de l'impulsion est ajusté en modifiant la dispersion du signal émis à l'aide d'un élément dispersif (réseaux de Bragg volumique ou fibré, miroir à dispersif, prisme).

Dans le domaine spectral, le dispositif de source laser 1 utilisé pour le traitement génère plusieurs longueurs d'onde discrètes ou est continument accordable sur une large bande dans les domaines du visible et/ou de l'infrarouge. Cela s'opère en insérant un filtre au sein de la cavité laser pour le rayonnement infra rouge. Le rayonnement visible est généré par doublage de fréquence.

Un dispositif de rétroaction 80 est disposé entre le dispositif de photodétection 50 et le dispositif de source laser 10 de traitement. Un processeur de calcul analyse en temps réel les mesures d'absorption du tissu 8 afin de déterminer la longueur d'onde adaptée à la peau ou au tatouage. L'information analysée est ensuite transmise au logiciel de pilotage du dispositif de source laser 1 qui adapté la longueur d'onde en fonction du pic d'absorption du chromophore, du tissu ou du tatouage à traiter. De façon alternative, la sélection de la longueur d'onde de traitement est effectuée avant le traitement. A cet effet, on acquiert par exemple une image spectrale de la zone à traiter, avec un enregistrement des différentes longueurs d'onde à fournir pour le laser de traitement en fonction de chaque point de l'image.

### Procédé

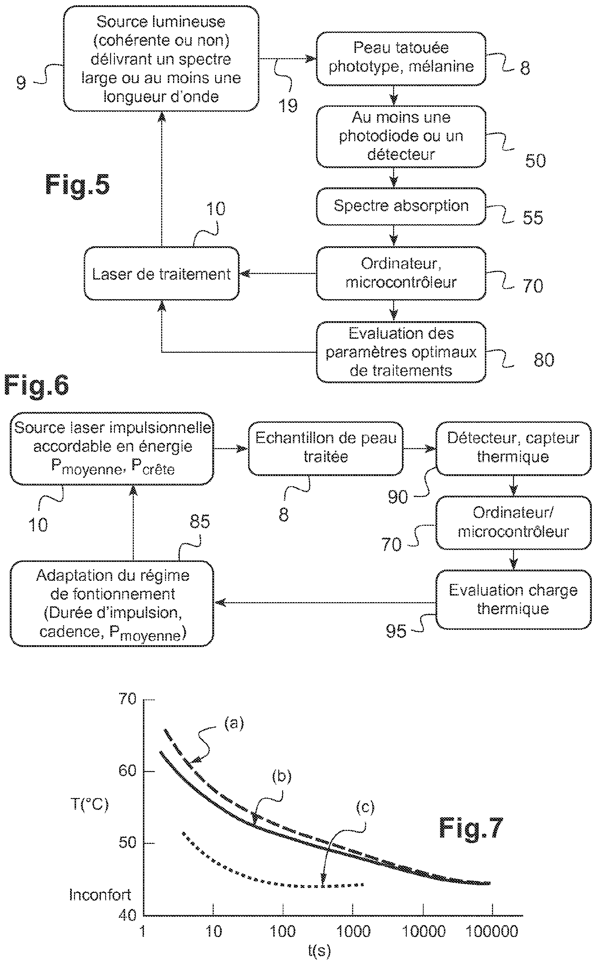

Sur la figure 5, est représenté un exemple de système et procédé de traitement laser pour la dermatologie. Dans une première étape, une autre source lumineuse 9 génère un faisceau lumineux 19 de faible intensité en direction de la peau 8 à analyser. Le système de photo-détection 5 et de spectrométrie 6 mesure un spectre de réflexion de la peau 8. Un calculateur en déduit un spectre d'absorption 55 de la peau 8. Un ordinateur ou un micro-contrôleur 70 sélectionne une longueur d'onde, une durée (comprise entre 30 ps et 10 ns) et une cadence de répétition (entre 10 MHz et 500 MHz) du laser de traitement 10 en fonction du spectre d'absorption mesuré. On applique un train d'impulsion laser de traitement sur la peau 8.

L'autre source d'illumination 9 de l'échantillon de peau peut être une source cohérente ou non cohérente émettant un spectre large ou au moins une longueur d'onde discrète. Cette autre source lumineuse 9 peut être le laser 10 de traitement mais configuré pour délivrer un faisceau lumineux 19 peu puissant et/ou peu énergétique afin de ne pas dégrader ou altérer l'échantillon de peau. Le faisceau lumineux 19 éclaire la peau 8 à analyser et à traiter. Le faisceau réfléchi et/ou rétro-diffusé est collecté au moyen du dispositif de collecte et mesuré au moyen du dispositif de spectrométrie 6 et de photodétection 5 comprenant une ou plusieurs photodiodes. Les variations des mesures d'absorption (déduites des mesures de réflexion et/ou rétro-diffusion) sont enregistrées. On en déduit une ou plusieurs bandes spectrales d'absorption principalement par la peau 8. Ces bandes spectrales d'absorption correspondent aux différentes couleurs d'un tatouage, à la mélanine de la peau et/ou à des marqueurs utilisés en photothérapie dynamique (PDT). Les conditions de traitement laser optimales sont déduites de cette analyse.

Une boucle de rétroaction 80 est alors couplée avec le dispositif de source laser de traitement 10. Le dispositif de source laser de traitement 10 comprend un laser accordable en longueur d'onde dans le domaine spectral du visible et de l'infra-rouge. De façon alternative, le dispositif de source laser de traitement 10 comporte plusieurs lasers mono-longueurs d'onde dans le domaine spectral du visible et de l'infra-rouge. Ces lasers sont des lasers continus ou à impulsions en fonction des applications. De préférence, le dispositif de source laser de traitement 10 émet des impulsions laser de durée comprise entre 500 femtoseconde et 100 microseconde avec des cadences allant de 1 Hz jusqu'à 1 GHz. Le faisceau laser de traitement a une puissance moyenne comprise entre 100 mW et 100 W.

Ce système de traitement laser asservi en temps réel permet d'effectuer de nombreux traitements en dermatologie comme l'enlèvement de tatouage, l'épilation, le photo-rajeunissement ou la photothérapie dynamique.

Ce système de traitement laser permet également de déterminer en temps réel l'avancée du traitement grâce à l'amplitude du signal collecté lié à l'absorption de la surface. Par exemple, dans un procédé de détatouage, plus la concentration en pigment diminue plus l'amplitude du signal collecté d'absorption est faible. Il est ainsi possible d'arrêter le traitement laser grâce à une mesure directe in situ et non basée sur des simulations prédisant les effets du traitement.

En pratique, le spectre d'absorption de la peau peut varier énormément d'un patient à l'autre en fonction de son phototype. De plus, d'une peau à l'autre les coefficients de diffusivité thermique et de conductivité thermique varient aussi. Par conséquent, les effets thermiques induits par un même traitement peuvent varier d'un patient à un autre.

Pour contrôler ces effets thermiques, la figure 6 illustre une variante d'un système de traitement laser. Ce système comporte une source laser à impulsions accordable en durée et fréquence de répétition pour effectuer un traitement laser thermiquement contrôlé.

Ce type de fonctionnement temporel présente un intérêt particulièrement innovant en dermatologie, ou la combinaison puissance crête ET puissance moyenne dans l'impulsion relativement importantes ouvre la voie à une nouvelle sorte d'interaction dans laquelle les effets photo-ablatifs couplés à des effets thermiques peuvent jouer des rôles complémentaires.

Le système de la figure 6 comporte une source laser 10 accordable en en fréquence de répétition. La source laser 10 génère des impulsions laser ou des paquets d'impulsions. Ce système est basé sur une architecture laser dite à mode bloqué. Le régime mode bloqué peut être obtenu par l'utilisation d'un absorbant saturable, d'un modulateur ou de rotation non linéaire de la polarisation. L'association de cette source à un élément permettant d'extraire une ou plusieurs impulsions (modulateur acousto optique ou electro-optique, cellule de pockels) génère une impulsion unique ou des paquets de plusieurs impulsions.

Le système de traitement laser comporte ici un capteur thermique 90 incorporé dans la tête du laser en contact avec l'épiderme. Le capteur thermique 90 mesure la température sur la peau traitée. Cette information permet d'adapter la charge thermique apportée au patient au cours du traitement laser afin d'éviter les lésions engendrées par une température et/ou une durée d'exposition trop élevées.

En effet, la figure 7 montre une courbe (a) représentative de l'apparition de brûlures profondes, respectivement la courbe (b) de brûlures superficielles et la courbe (c) d'un inconfort, en fonction de la durée d'exposition au traitement laser et de la température en surface de la peau.

## Revendications

1. Système de diagnostic et de traitement dermatologique comprenant :
- un dispositif de source lumineuse (2),
- un dispositif de collecte (4) de flux lumineux,
- un dispositif de mesure spectroscopique (5, 6) d'absorption, et
- un calculateur,
**caractérisé en ce que** :
- le dispositif de source lumineuse (2) est adapté pour générer un premier faisceau lumineux (20) de faible intensité en direction d'une surface épidermique (8) d'un patient, le premier faisceau lumineux (20) s'étendant sur une gamme spectrale ou étant accordable en longueur d'onde sur une gamme spectrale,
- le dispositif de collecte (4) de flux lumineux étant arrangé pour collecter un premier flux lumineux par réflexion et/ou diffusion du premier faisceau lumineux (20) sur ladite surface épidermique (8),
- le dispositif de mesure spectroscopique (5, 6) d'absorption est adapté pour enregistrer une première mesure spectroscopique du premier flux lumineux en fonction de la gamme spectrale, et
- le calculateur étant adapté pour recevoir la première mesure spectroscopique dans la gamme spectrale et pour déterminer une première longueur d'onde associée à une absorption d'un pigment de ladite surface épidermique et une deuxième longueur d'onde associée à une absorption d'un autre constituant tissulaire de ladite surface épidermique, et **en ce que**
- le dispositif de source lumineuse comporte une source laser (10) accordable en longueur d'onde à l'intérieur de la gamme spectrale, la source laser étant accordable en durée et/ou en cadence et la source laser étant adaptée pour générer un deuxième faisceau laser (1) de traitement de forte intensité à ladite première longueur d'onde en direction de ladite surface épidermique (8) et **en ce que** le système de diagnostic et de traitement dermatologique comporte en outre un capteur de température et un dispositif de rétroaction (80, 90, 95), le capteur de température étant arrangé pour enregistrer un signal de mesure de température de ladite surface épidermique en fonction de l'application du deuxième faisceau laser et le dispositif de rétroaction (80, 90, 95) étant configuré pour modifier la durée et/ou la cadence des impulsions laser en fonction du signal de mesure de température.

2. Système de diagnostic et de traitement dermatologique selon la revendication 1 dans lequel le deuxième faisceau laser (1) est constitué d'impulsions de durée comprise entre 30 ps et 10 ns.

3. Système de diagnostic et de traitement dermatologique selon l'une des revendications 1 à 3 dans lequel le dispositif de collecte (4) de flux lumineux comporte une sphère intégrante, et dans lequel le dispositif de mesure spectroscopique (5, 6) d'absorption comporte un dispositif de photo-détection (5) et un spectromètre ou un monochromateur.

4. Système de diagnostic et de traitement dermatologique selon l'une des revendications 1 à 3 dans lequel la source laser (10) est continument accordable en longueur d'onde sur une gamme spectrale s'étendant au moins sur 100 nm.

5. Système de diagnostic et de traitement dermatologique selon la revendication 4 dans lequel la source laser (10) est adaptée pour générer le premier faisceau lumineux (20) et le deuxième faisceau laser (1) et dans lequel le dispositif de source lumineuse (2) comporte en outre un dispositif de modulation en intensité du faisceau laser généré par la source laser.

6. Système de diagnostic et de traitement dermatologique selon l'une des revendications 1 à 5 dans lequel le dispositif de source lumineuse (2) comporte une autre source lumineuse adaptée pour générer le premier faisceau lumineux (20).

7. Système de diagnostic et de traitement dermatologique selon l'une des revendications 1 à 6 dans lequel le dispositif de collecte (4) de flux lumineux est configuré pour collecter un deuxième flux lumineux (50) par réflexion et/ou diffusion du deuxième faisceau lumineux (1) sur ladite surface épidermique (8) et dans lequel le dispositif de mesure spectroscopique (5, 6) d'absorption est adapté pour enregistrer un signal de mesure du deuxième flux lumineux à la première longueur d'onde.

8. Système de diagnostic et de traitement dermatologique selon la revendication 7 dans lequel le dispositif de rétroaction (80, 90, 95) est configuré pour modifier la durée et/ou la cadence des impulsions laser en fonction du signal de mesure du deuxième flux lumineux.

9. Système de diagnostic et de traitement dermatologique selon l'une des revendications 1 à 8 dans lequel le dispositif de mesure spectroscopique comporte un détecteur d'image ayant une matrice de pixels adaptée pour former une image hyperspectrale de ladite surface épidermique dans ladite gamme spectrale, et le calculateur étant adapté pour déterminer la deuxième longueur d'onde de traitement laser correspondant à chaque pixel du détecteur d'image à partir de ladite image hyperspectrale.

## Patentansprüche

1. Dermatologisches Diagnose- und Behandlungssystem mit
- einer Lichtquellenvorrichtung (2),
- einer Vorrichtung (4) zum Sammeln von Lichtfluß,
- einer Vorrichtung (5, 6) zum spektroskopischen Messen von Absorption und
- einem Rechner,
**dadurch gekennzeichnet, daß**
- die Lichtquellenvorrichtung (2) dazu ausgelegt ist, einen ersten Lichtstrahl (20) geringer Stärke auf eine Hautoberfläche (8) eines Patienten zu richten, wobei sich der erste Lichtstrahl (20) über einen Spektralbereich erstreckt oder über einen Spektralbereich hinsichtlich seiner Wellenlänge abstimmbar ist,
- die Vorrichtung (4) zum Sammeln von Lichtfluß dazu ausgelegt ist, einen ersten Lichtfluß durch Reflexion und/oder Streuung des ersten Lichtstrahls (20) an der Hautoberfläche (8) zu sammeln,
- die Vorrichtung (5, 6) zum spektroskopischen Messen von Absorption dazu ausgelegt ist, eine erste spektroskopische Messung des ersten Lichtflusses in Abhängigkeit vom Spektralbereich einzuspeichern, und
- der Rechner dazu ausgelegt ist, die erste spektroskopische Messung im Spektralbereich zu empfangen und eine mit einer Absorption eines Pigments der Hautoberfläche verbundene erste Wellenlänge und eine mit einer Absorption eines weiteren Gewebebestandteils der Hautoberfläche verbundene zweite Wellenlänge zu bestimmen, und daß
- die Lichtquellenvorrichtung eine innerhalb des Spektralbereichs wellenlängenmäßig abstimmbare Laserquelle (10) aufweist, wobei die Laserquelle hinsichtlich der Dauer und/oder der Kadenz abstimmbar ist und die Laserquelle dazu ausgelegt ist, einen zweiten Laserstrahl (1) großer Stärke zur Behandlung bei der ersten Wellenlänge in Richtung auf die Hautoberfläche (8) zu erzeugen, und daß das dermatologische Diagnose- und Behandlungssystem außerdem einen Temperatursensor und eine Rückkopplungsvorrichtung (80, 90, 95) aufweist, wobei der Temperatursensor dazu ausgelegt ist, ein Temperaturmeßsignal der Hautoberfläche in Abhängigkeit von der Anwendung des zweiten Laserstrahls einzuspeichern, und die Rückkopplungsvorrichtung (80, 90, 95) dazu ausgelegt ist, die Dauer und/oder die Kadenz der Laserimpulse in Abhängigkeit vom Temperaturmeßsignal zu modifizieren.

2. Dermatologisches Diagnose- und Behandlungssystem gemäß Anspruch 1, bei dem der zweite Laserstrahl (1) aus Impulsen mit einer Dauer zwischen 30 ps und 10 ns gebildet ist.

3. Dermatologisches Diagnose- und Behandlungssystem gemäß einem der Ansprüche 1 bis 2, bei dem die Vorrichtung (4) zum Sammeln von Lichtfluß eine Ulbricht-Kugel aufweist und bei dem die Vorrichtung (5, 6) zum spektroskopischen Messen von Absorption eine Lichterfassungsvorrichtung (5) und ein Spektrometer oder einen Monochromator aufweist.

4. Dermatologisches Diagnose- und Behandlungssystem gemäß einem der Ansprüche 1 bis 3, bei dem die Laserquelle (10) wellenlängenmäßig über einen sich über wenigstens 100 nm erstreckenden Spektralbereich durchgehend abstimmbar ist.

5. Dermatologisches Diagnose- und Behandlungssystem gemäß Anspruch 4, bei dem die Laserquelle (10) dazu ausgelegt ist, den ersten Lichtstrahl (20) und den zweiten Laserstrahl (1) zu erzeugen, und bei dem die Lichtquellenvorrichtung (2) außerdem eine Vorrichtung zur Modulation der Stärke des durch die Laserquelle erzeugten Laserstrahls aufweist.

6. Dermatologisches Diagnose- und Behandlungssystem gemäß einem der Ansprüche 1 bis 5, bei dem die Lichtquellenvorrichtung (2) eine weitere Lichtquelle aufweist, die dazu ausgelegt ist, den ersten Lichtstrahl (20) zu erzeugen.

7. Dermatologisches Diagnose- und Behandlungssystem gemäß einem der Ansprüche 1 bis 6, bei dem die Vorrichtung (4) zum Sammeln von Lichtfluß dazu ausgelegt ist, einen zweiten Lichtfluß (50) durch Reflexion und/oder Streuung des zweiten Lichtstrahls (1) auf der Hautoberfläche (8) zu sammeln, und bei dem die Vorrichtung (5, 6) zum spektroskopischen Messen von Absorption dazu ausgelegt ist, ein Meßsignal des zweiten Lichtflusses bei der ersten Wellenlänge einzuspeichern.

8. Dermatologisches Diagnose- und Behandlungssystem gemäß Anspruch 7, bei dem die Rückkopplungsvorrichtung (80, 90, 95) dazu ausgelegt ist, die Dauer und/oder die Kadenz der Laserimpulse in Abhängigkeit vom Meßsignal des zweiten Lichtflusses zu modifizieren.

9. Dermatologisches Diagnose- und Behandlungssystem gemäß einem der Ansprüche 1 bis 8, bei dem die Vorrichtung zum spektroskopischen Messen einen Bildsensor mit einer Pixelmatrix aufweist, die dazu ausgelegt ist, ein hyperspektrales Bild der Hautoberfläche im Spektralbereich zu bilden, und wobei der Rechner dazu ausgelegt ist, vom hyperspektralen Bild ausgehend, die jedem Pixel des Bildsensors entsprechende zweite Wellenlänge zur Laserbehandlung zu bestimmen.

## Claims

1. A dermatological diagnostic and treatment system comprising:
- a light source device (2),
- a light flow collecting device (4),
- an absorption spectroscopic measurement device (5, 6), and
- a calculator,
**characterized in that**:
- the light source device (2) is adapted to generate a first low-intensity light beam (20) towards an epidermal surface (8) of a patient, the first light beam (20) extending over a spectral range or being tunable in wavelength over a spectral range,
- the light flow collecting device (4) being arranged to collect a first light flow by reflection and/or scattering of the first light beam (20) on said epidermal surface (8),
- the absorption spectroscopic measurement device (5, 6) is adapted to record a first spectroscopic measurement of the first light flow as a function of the spectral range, and
- the calculator being adapted to receive the first spectroscopic measurement in the spectral range and to determine a first wavelength associated with an absorption of a pigment of said epidermal surface and a second wavelength associated with an absorption of another tissue constituent of said epidermal surface, and **in that**
- the light source device includes a laser source (10) tunable in wavelength inside the spectral range, the laser source being tunable in duration and/or rate and the laser source being adapted to generate a second treatment laser beam (1) of high intensity at said first wavelength towards said epidermal surface (8) and **in that** the dermatological diagnostic and treatment system further includes a temperature sensor and a feedback device (80, 90, 95), the temperature sensor being arranged to record a temperature measurement signal of said epidermal surface as a function of the application of the second laser beam and the feedback device (80, 90, 95) being configured to modify the duration and/or rate of the laser pulses as a function of the temperature measurement signal.

2. The dermatological diagnostic and treatment system according to claim 1, wherein the second laser beam (1) is consisted of pulses of duration comprised between 30 ps and 10 ns.

3. The dermatological diagnostic and treatment system according to one of claims 1 to 3, wherein the light flow collecting device (4) includes an integrating sphere, and wherein the absorption spectroscopic measurement device (5, 6) includes a photo-detection device (5) and a spectrometer or a monochromator.

4. The dermatological diagnostic and treatment system according to one of claims 1 to 3, wherein the laser source (10) is continuously tunable in wavelength over a spectral range extending over at least 100 nm.

5. The dermatological diagnostic and treatment system according to claim 4, wherein the laser source (10) is adapted to generate the first light beam (20) and the second laser beam (1) and wherein the light source device (2) further includes a device for intensity modulating the laser beam generated by the laser source.

6. The dermatological diagnostic and treatment system according to one of claims 1 to 5, wherein the light source device (2) includes another light source adapted to generate the first light beam (20).

7. The dermatological diagnostic and treatment system according to one of claims 1 to 6, wherein the light flow collecting device (4) is configured to collect a second light flow (50) by reflection and/or scattering of the second light beam (1) on said epidermal surface (8) and wherein the absorption spectroscopic measurement device (5, 6) is adapted to record a second light flow measurement signal at the first wavelength.

8. The dermatological diagnostic and treatment system according to claim 7, wherein the feedback device (80, 90, 95) is configured to modify the duration and/or the rate of the laser pulses as a function of the second light flow measurement signal.

9. The dermatological diagnostic and treatment system according to one of claims 1 to 8, wherein the spectroscopic measurement device includes an image detector having a pixel array adapted to form a hyperspectral image of said epidermal surface in said spectral range, and the calculator being adapted to determine the second laser treatment wavelength corresponding to each pixel of the image detector from said hyperspectral image.
